(19) Europäisches Patentamt
European Patent Office
Office européen des brevets

(11) Publication number: **0 333 078 B1**

(12) **EUROPEAN PATENT SPECIFICATION**

(45) Date of publication of patent specification: **17.02.93**

(51) Int. Cl.[5]: **C07C 43/04**, C07C 41/09, B01J 21/04, B01J 21/08

(21) Application number: **89104334.1**

(22) Date of filing: **11.03.89**

(54) **Method for one-step synthesis of methyl t-butyl ether.**

(30) Priority: **14.03.88 US 168063**

(43) Date of publication of application:
**20.09.89 Bulletin 89/38**

(45) Publication of the grant of the patent:
**17.02.93 Bulletin 93/07**

(84) Designated Contracting States:
**DE ES FR GB IT NL**

(56) References cited:
**EP-A- 0 129 842**
**GB-A- 403 402**
**US-A- 2 282 469**
**US-A- 4 144 138**
**US-A- 4 337 366**

**CHEMICAL ABSTRACTS, vol. 92, no. 7, 18 February 1980, Columbus, Ohio, USA; S. V. ROZHKOV et al, "Preparation of methyl tert.-butyl ether from methyl and tert.-butyl alcohols in the presence of cation exchanges", page 619, abstract no. 58 165y**

(73) Proprietor: **TEXACO DEVELOPMENT CORPORATION**
**2000 Westchester Avenue**
**White Plains, New York 10650(US)**

(72) Inventor: **Knifton, John Frederick**
**10900 Catskill Trail**
**Austin Texas 78750(US)**

(74) Representative: **Brock, Peter William et al**
**UROUHART-DYKES & LORD 91 Wimpole Street**
**London W1M 8AH (GB)**

EP 0 333 078 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention concerns an improved process for preparing methyl tertiary butyl ether by the reaction of tertiary butanol and methanol in the presence of a catalyst containing alumina, silica and/or zeolites, particularly super-acid aluminas and silica-rich zeolites. Also useful are acidic clay or clay mineral catalysts containing alumina or silica, such as smectite clays, including acidic montmorillonite silica-alumina clays. The invention is particularly advantageous in that the reaction takes place in one-step, the catalyst exhibits excellent selectivity to the desired ether product and high levels of tert-butanol conversion are achieved.

It is known to those skilled in the art that ethers, including unsymmetrical ethers, may be prepared by reacting an alcohol with another alcohol to form the desired product. The reaction mixture, containing catalyst and/or condensing agent may be separated and further treated to permit attainment of the desired product. Such further treatment commonly includes one or more distillation operations.

Methyl tert-butyl ether is finding increasing use as a blending component in high octane gasoline as the current gasoline additives based on lead and manganese are phased out. Currently all commercial processes for the manufacture of methyl tert-butyl ether (MTBE) are based upon the liquid-phase reaction of isobutylene and methanol (eq. 1), catalyzed by a cationic ion-exchange resin (see, for example: hydrocarbon processing, Oct. 1984, p. 63; Oil and Gas J., Jan. 1, 1979, p. 76; Chem. Economics Handbook-SRI, Sept. 1986, p. 543-705:P). The cationic ion-exchange resins used in MTBE synthesis normally have the sulphonic acid functionality (see: J. Tejero, J. Mol. Catal., 42 (1987) 257; C. Subramamam et al., Can. J. Chem. Eng., 65 (1987) 613).

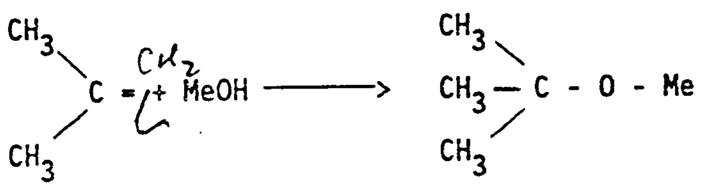

(Eq. 1)

With the expanding use of MTBE as an acceptable gasoline additive, however, a growing problem is the availability of raw materials. Historically, the critical raw material is isobutylene (Oil and GAS J., June 8, 1987, p. 55). It would be advantageous, therefore, to have a process to make MTBE that does not require isobutylene as a building block. It would be advantageous to have an efficient process for making MTBE by reaction of methanol with tertiary butyl alcohol, since t-butanol (TBA) is readily available commercially through isobutane oxidation.

In U. S. Patent No. 4,144,138 (1979) to Rao et al., there is disclosed a method for recovering methyl tertiary butyl ether from etherification reaction effluent by azeotropic distillation to recover methanol-ether azeotrope overhead which is water-washed to give pure ether raffinate, the latter being azeotropically distilled to yield ether-methanol overhead which is recycled to water washing.

The preparation of methyl tert-butyl ether from methyl and tert-butyl alcohols is discussed in S. V. Rozhkov et al., Prevrashch Uglevodorodov, Kislotno-Osnovn. Geterogennykh Katal. Tezisy Dokl. Vses Konf., 1977, 150 (C. A. 92:58165y). Here the TBA and methanol undergo etherification over KU-2 strongly acidic sulfopolystyrene cation-exchangers under mild conditions. This reference contains data on basic parameters of such a process. It is also pointed out that, although a plant for etherification over cation exchangers does not present any problems, considerations include the fact that recycling large amounts of tert-butyl alcohol and methanol, as well as isobutylene, causes the scheme to be somewhat more expensive. Also, the progress of the reaction over cation exchangers is usually complicated by various adsorption and diffusion factors, by swelling phenomena, and by the variable distribution of the components between the solution and ion-exchanger phase. Furthermore, said acidic cation-exchangers with an organic (polystyrene or polymethacrylate) backbone generally have a very limited stability range with regard to operating temperatures, with temperatures above 120 ° C normally leading to irreversible destruction of the resin and loss of catalytic activity.

US-A-2282469 discloses the preparation of ethers by passing alcohols at elevated temperature over a hydration acid catalyst, such as phosphoric, sulphuric or persulphuric acid, deposited on a support such as Kieselguhr, pumice or alumina.

US-A-4337366 discloses that active alumina reacted with certain silicon compounds (such as tetraalkyl orthosilicates) can be used as a catalyst in the conversion of alcohols into ethers.

In an article titled "Catalysis: Selective Developments", Chem. Systems Report 84-3, 239-249, at section 3.4320, the unusual properties of smectite clays which make them of interest as catalysts are discussed. These compositions are layered and exhibit a 2:1 relationship between tetrahedral and octahedral sites. In addition the combination of cation exchange, intercalation and the fact that the distance between the layers can be adjusted provide interesting possibilities.

There is a discussion of clay mineral catalysts, including "acid" montmorillonite clay catalysts in "Progress in Inorganic Chemistry", Vol. 35, p. 41 (1987). The process of pillaring this type of catalyst is discussed. Pillaring can convert a clay lamellar solid into a more heat resistant two dimensional zeolite material.

G. B. Patent No. 2,179,563 (1987) discloses the use of modified layered clay catalysts in reactions capable of catalysis by protons. Of particular interest in this invention were the three-layer sheet types, such as smectites, micas and vermiculites composed of successive layers of tetrahedral silica, octahedral alumina and tetrahedral silica which can exhibit swelling properties.

It would be a substantial advance in the art if methyl tertiary butyl ether could be selectively synthesized from tertiary butyl alcohol and methanol in one step using an alumina or silica rich zeolite or clay mineral catalyst which allows for rapid conversion of t-butanol.

In accordance with certain of its aspects, the novel method of this invention for preparing methyl tert-butyl ether from tertiary butyl alcohol (t-butanol) and methanol in one-step comprises reacting tertiary butyl alcohol and methanol in the presence of a catalyst comprising an alumina, or silica, or a zeolite containing alumina and/or silica, or a clay mineral catalyst containing alumina and/or silica, at an elevated temperature and moderate pressure. Examples demonstrate the effectiveness of super acid aluminas and silica-rich zeolites and smectite clays, including acidic montmorillonite silica-alumina clays.

Preparation of the product of this invention may be carried out typically by reacting tertiary butyl alcohol and methanol in the presence of an etherification catalyst. The etherification is carried out in one step and the catalyst preferably comprises an acidic alumina, acidic silica, silica-alumina or an acidic zeolite containing silica and/or alumina, or an acidic silica-alumina clay catalyst.

The reaction can be represented by the following:

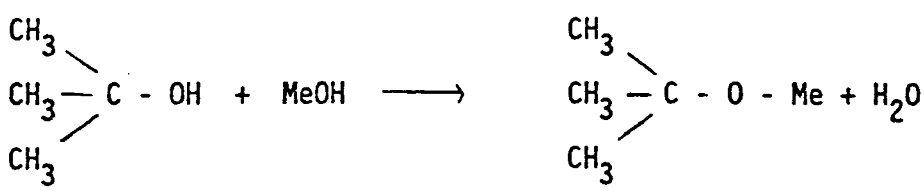

(Eq. 2)

Generally the methanol and t-butanol coreactants may be mixed in any proportion in order to generate the desired methyl t-butyl ether, but preferably the molar ratio of methanol to t-butanol in the feed mixture should be between 10:1 and 1:10, if the yield of desired MTBE is to be maximized. In order to achieve maximum selectivity to MTBE, and optimum conversion per pass, an excess of methanol in the liquid feed is desirable. The most preferred methanol-to-tertiary butanol molar ratio is from 1:1 to 5:1.

The catalysts used to effect this reaction are aluminas, silicas, silica-aluminas, or silica rich zeolites and silica-alumina clay catalysts.

In the case where the catalyst is alumina, silica or silica-alumina the catalysts are in the form of solids containing at least about 10 wt% alumina, silica or a combination thereof.

While a variety of alumina catalysts may be effective in the subject reaction (Eq. 2), it is necessary only that the alumina be acidic under normal operating conditions. The acidic sites on the solid alumina matrix may be either Lewis or Bronsted acid sites, or combinations thereof. The alumina may take many different forms, it may be an $\alpha$-alumina, a $\beta$-alumina, or any type of $\gamma$ -alumina, including transitional forms (see industrial alumina chemical, by C. Misra, ACS Monogram 184). The identification of surface acidity of such aluminas may be confirmed by titration with amine base, e.g. ammonia, n-butylamine, etc.

Said aluminas may or may not contain impurities, such as the alkali metals, e.g. sodium or potassium, or alkaline earth metals such as calcium.

The preferred alumina catalysts are 'super' acid aluminas, that are believed to contain both Bronsted and Lewis acid sites, and which have a surface area of greater than 10 $m^2/g$, especially >100 $m^2/g$. Such super acid aluminas include the super acid aluminas marketed by Harshaw-Filtrol Partnership, such as Harshaw-Filtrol Super Aluminas AL-3998, and AL-4198. These super acid aluminas have a greater proportion of Bronsted-acid-to-Lewis acid sites on the available surface than are normally found with standard aluminas.

Good results were also obtained using zeolites. The zeolites which are catalysts in the subject reaction comprise a group of crystalline aminosilicate structures wherein the structural formula of the zeolite is based on the crystal unit cell of which the smallest unit of the structure is represented by:

$M_{x/n}[(AlO_2)_x(SiO_2)_y].wH_2O$

where n is the valence of cation M, w is the number of water molecules per unit cell, x and y are the total number of tetrahedra per unit cell and y/x usually has values of 1-5.

In most zeolite structures the primary structural units are tetrahedra and these are assembled into secondary building units which may be simple polyhedra such as cubes, hexagonal prisms or octahedra. The final structural framework consists of assemblages of the secondary units.

Zeolites which can be used include synthetic zeolites A, X, Y and Zeolon H. In addition, Pentasil, high silica synthetic zeolites are also useful such as ZSM-5 and ZSM-11. Also effective are natural zeolites such as erionite, faujasite, mordenite, offertite, and chabazite.

Preferable said zeolites should be in a strongly acidic form whereby some, or all, of the cation M (usually a Group I or II, alkali or alkaline earth metal ion such as sodium, potassium or calcium) is exchanged by protons through mineral acid (HCl) treatment, etc. Calcination at elevated temperatures (e.g. 400 ° C) may also provide a particularly protonated material containing strong acid sites.

Typical formulas for the synthetic zeolites are given in the following table:

TABLE 1

| Synthetic Zeolites | Typical formula |
|---|---|
| Zeolite A | $Na_{12}[(AlO_2)_{12}(SiO_2)_{12}].27H_2O$ |
| Zeolite X | $Na_{86}[(AlO_2)_{86}(SiO_2)_{106}].264H_2O$ |
| Zeolite Y | $Na_{56}[(AlO_2)_{56}(SiO)_{136}].250H_2O$ |
| Zeolite L | $K_9[(AlO)_2)_9(SiO_2)_{27}].22H_2O$ |
| Zeolite Omega | $Na_{6.8}TMA_{1.6}[AlO_2)_8(SiO_2)_{28}].21H_2O^a$ |
| 25M-5 | $(Na,TPA)_3[(AlO_2)_3(SiO_2)_{93}].16H_2O^b$ |

[a]TMA = tetramethylammonium
[b]TPA = tetrapropylammonium
1. 15 Kirk-Othmer Encylopedia of Chemical Technology 639 (1981)

Illustrative of suitable zeolites for the one-step synthesis of MTBE from methanol plus t-butanol include Y-zeolites such as United Catalyst's Zeolite-Y, Z6-06-02, dealuminized Y-zeolites such as Zeochem's Zeolite-Y, L-2585, high silica zeolites, such as silicalite from Union Carbide, as well as transition-metal treated zeolites, such as a nickel-treated Zeolite-Y. The performance of such zeolites in MTBE synthesis (Eq. 2) is illustrated in the accompanying Examples I, II, V and VI.

Said catalysts may be in the form of powders, pellets, granules, spheres, shapes and extrudates. The examples described herein demonstrate the advantages of using granules and extrudates. Extrudates which work well include Y-zeolite extrudates (#Z6-06-02) from United Catalysts, extrudates with a surface area of 450 $m^2/g$. Another zeolite, used in Example II is dealuminized Y-zeolite from Zeochem Company, a powder with a surface area of >100 $m^2/g$.

A super acid alumina was used in Example III which has a high pore volume (#583A-22-16.6) from Harshaw, 3 mm (E-1/8") extrudate with a surface area of 190 $m^2/g$. Also used in Example IV was #583A-22-15-9, a super acid alumina from Harshaw with 3 mm (E-1/8") and surface of 175 $m^2/g$.

As will be demonstrated by the examples, these catalysts are preferably of high purity and high surface area. It has been found in the process of this invention that greater conversion of tertiary butanol and methanol is achieved where the surface area of the support catalyst is generally >10 $m^2/g$.

As discussed, another group of catalysts which works well in this synthesis are acidic clay mineral catalysts. Chemically, clays are composed primarily of silicon, aluminum and oxygen, with minor amounts of magnesium and iron in some cases. Variations in the ratios of these consistuents, and their crystal lattice configurations, results in some fifty separate clays, each with its own characteristic properties.

Particularly effective in reaction (Eq. 2) are smectite clays. Smectite clays are discussed in the article cited in Chem. Systems Report, 84-3. These clays have small particle size and unusual intercalation

properties which afford them high surface area. They are alumino silicates with a unique structure that permits modifications which should provide useful catalysts.
They comprise layered sheets of octahedral sites between sheets of tetrahedral sites, where the distance between the layers can be adjusted by swelling. What renders the smectites of interest among the clay minerals is the combination of cation exchange, intercalation, and the fact that the distance between the layers can be adjusted by treatment with the appropriate solvent etc.

The three layered sheet types include montmorillonite, vermiculite and some brittle mica. The idealized basic structure of clays of this type is that of a pyrophyllite which has the basic formula $Si_8Al_4O_{20}(OH)_4$.

A general representation of the montmorillonite structure is:

$$M_{x/n}^{n+}\text{-}yH_2O(Al_{4-x}Mg_x)(Si_8)O_{20}(OH)_4$$

Where: M represents the interlamellar (balancing cations), normally sodium or lithium. x, y and n are integers.

Said montmorillonite clays are best used in the present application in an acidic form. Acids activate montmorillonites by attacking and solubilizing structural cations in the octahedral layers. This opens up the clay structure and increases surface area. These acid treated clays act as strong Bronsted acids.

Acidic montmorillonite clays are the preferred form of mineral clay in the present invention. Illustrative examples include Harshaw-Filtrol Clays-113 and-13, in powder form, the granular Clay-24 as well as extruded Clay-62.

The reaction may be carried out in either a stirred slurry reactor or in a fixed bed continuous flow reactor. The catalyst concentration should be sufficient to provide the desired catalytic effect.

Etherification can generally be conducted at temperatures from 20° to 250°C; the preferred range is 80° to 180°C. The total operating pressure may be from atmospheric to 6995 kPa (0 to 1000 psig), or higher. The preferred pressure range is 445 to 3547 kPa (50 to 500 psig).

Typically, MTBE is generated continuously in up to ca. 44 wt% concentration in the crude liquid product at total liquid hourly space velocities (LHSV) of up to 10 and relatively mild conditions, where:

$$\text{LHSV} = \frac{\text{Volume Of Total Liquid Feed Run Through The Reactor Per Hour}}{\text{Volume of Catalyst In Reactor}}$$

The examples which follow illustrate the one-step synthesis of MTBE from TBA and MeOH (Eq. 2) using zeolites and aluminas and clay mineral catalysts, particularly acidic clay catalysts, silica-rich zeolites and high acid aluminas in the form of high surface area extrudates. The examples are only intended as a means of illustration and it is understood the invention is not meant to be limited thereby.

Conversions of t-butanol (TBA, wt%) are estimated in the following examples using the equation:

$$\frac{(\text{Wt\% Conc. of TBA in Feed} - \text{Wt\% Conc. of TBA in Product})}{\text{Wt\% Conc. of TBA in Feed}} \times 100$$

Yields of methyl t-butyl ether (MTBE, mole %) are estimated from:

$$\frac{\text{Moles of MTBE in Product Liquid}}{\text{moles of TBA converted}} \times 100$$

It may be noted that in Example I, United Catalysts Y-Zeolite, Z6-06-02 gave MTBE in ca. 39% concentration when run at LHSV of 1 (e.g. Sample #17) and ca. 34% concentration in the crude liquid

product when run at LHSV of 4 (e.g. Sample #22). The operating conditions in both cases (140 ° C, 2168 kPa [300 psig]) are moderate. This catalyst was screened over the temperature range of 100-140 ° C. At 140 ° C, LHSV = 4, Sample #22 shows:

Estimated TBA conversion per pass = 71%

MTBE yield (basis TBA converted) = 73 mole %.

A typical MTBE synthesis procedures is detailed below:

EXAMPLE 1

This example illustrates the co-synthesis of methyl t-butyl ether from t-butanol and methanol using a particular aluminosilicate zeolite catalyst.

The synthesis was conducted in a tubular reactor 14 mm (0.563") id; 305 mm (12") long, constructed of 316 stainless steel, operated upflow and mounted in a furnace, controllable to ±1.0 ° C and fitted with pumps allowing flow control to <±1 $cm^3$/h. The reactor was also fitted with a pressure regulating device and equipment for monitoring temperature, pressure and flow rate.

The reactor was charged at the beginning of the experiment with 25 $cm^3$ of United Catalyst Y-zeolite extrudates (#Z6-06-02). A screen of glass beads was placed at the top and bottom of the reactor to ensure the zeolite would remain in the middle portion.

The catalyst bed was first conditioned overnight by washing with methanol/t-butanol (2:1 mix) at 100 ° C, 300 psi back pressure and a liquid flow rate of 25 $cm^3$/h. The same solution of methanol (1281.6g, 40.0 mole) plus t-butanol (1482.4g), (20.0 mole) was then pumped through the catalyst bed at 25 cc/hr, while the reactor was held at 100 ° C, at total pressure of 2168 kPa (300 psi). Samples of product were taken periodically either by trapping in a dry ice cooled container, or by collecting on-stream (on-line) in a 316 ss bomb. Typical analyses data for samples taken under these conditions are summarized in Table I. Catalyst performance at other operating temperatures and liquid flow rates was also measured, after reaching equilibrium conditions overnight. Summary data for these runs are also given in Table I.

EXAMPLES II TO VII

Using the procedures and analyses methods of Example I, these examples illustrate the one-step synthesis of MTBE from methanol plus t-butanol (2:1 mix) at a pressure of 2168 kPa (300 psig) over a range of operating temperatures and space velocities, but with the following catalysts (25 $cm^3$ each):

a) A dealuminized Y-zeolite from Zeochem, L-2585, in powder form.

b) A high pore volume, super acid alumina, AL-4198, from Harshaw/Filtrol, in extruded form.

c) A second super acid alumina, AL-3998, from Harshaw/Filtrol, also in extruded form.

d) A nickel-on-zeolite catalyst from Zeochem, as extrudates.

e) A silica-rich zeolite, Silicalite, type S-115, from Union Carbide, in extruded form.

f) An alumina extrudate from American Cyanamid.

The results are summarized in Tables I to V. Of Note:

In Example II, dealuminized Y-Zeolite from Zeochem gave MTBE in ca. 28% concentration when run at LHSV of 1 (e.g. Sample #15) and ca. 24% concentration in the crude liquid product when run at LHSV of 4 (e.g. Sample #27). The screening conditions in this series of runs were 100-160 ° C, 2168 kPa (300 psi). At 160 ° C, LHSV = 4, Sample #27 shows:

Estimated TBA conversion per pass = 56%

MTBE yield (basis TBA converted) = 68 mole %.

In the runs in Examples III and IV, two super-acid aluminas from Harshaw were evaluated for MTBE production. At 180 ° C, Sample #23 shows ca. 20% concentration of MTBE in the crude liquid product. A similar result was obtained at the same conditions (180 ° C, 2168 kPa (300 psi), LHSV = 1) with the second super-acid alumina catalysts (see Sample #32).

In Example VI, silicalite, 1.5 mm (1/16") extrudates, performed well with both 2:1 and 1:1 methanol/t-butanol molar feed ratios. At 150 ° C using a LHSV of 1, the crude product effluent contains up to 38 wt% MTBE (see Sample #8). Generally the use of 1:1 feed helps to raise the level of tBA conversion, but the MTBE product concentration and yield are both lower, e.g.:

| | | |
|---|---|---|
| For Sample #24, (2:1) Feed: | vs | Sample #30, (1:1) Feed: |
| tBA Conv. = 47% | | 57% |
| MTBE Yield = 67% | | 41% |

In Example VII, at 180°C, the alumina catalyst shows an MTBE concentration of ca. 29 wt%, and for Sample #17:

Estimated tBA Conversion Per Pass = 90%

MTBE Yield = 51 mole %

TABLE I

| Example | Catalyst | Feed Sample | Flow Rate (cm³/h) | Temp. (°C) | Sample | MTBE | $C-C_4$ | MeOH | tBA | $H_2O$ |
|---|---|---|---|---|---|---|---|---|---|---|
| | | | | | | ← PRODUCT COMPOSITION (WT%) → | | | | |
| | | F | | | | | | 45.0 | 54.8 | |
| I | Y-Zeolite Z6-06-02[a] | | 25 | 100 | #1 | 13.0 | 0.9 | 40.6 | 41.7 | 3.7 |
| | | | | | #6 | 14.0 | 1.1 | 39.2 | 39.9 | 5.6 |
| | | | | | #7 | 13.7 | 1.3 | 40.1 | 40.7 | 4.1[c] |
| | | | " | 120 | #8 | 35.0 | 2.7 | 32.6 | 19.6 | 10.0 |
| | | | | | #12 | 35.0 | 2.5 | 32.5 | 19.6 | 9.8 |
| | | | " | 140 | #14 | 37.9 | 4.9 | 31.6 | 12.8 | 12.5 |
| | | | | | #17 | 38.6 | 5.5 | 30.1 | 12.7 | 12.0 |
| | | | 100 | " | #18 | 34.9 | 5.5 | 32.6 | 16.2 | 10.7 |
| | | | | | #20 | 35.1 | 5.1 | 32.7 | 16.4 | 10.7 |
| | | | | | #22 | 34.0 | 5.9 | 31.9 | 15.8 | 12.3[c] |
| | | F | | | | | | 47.0 | 53.0 | |
| II | Y-Zeolite L-2585[b] | | 25 | 100 | #1 | 3.7 | 1.3 | 45.4 | 48.7 | 0.7 |
| | | | | | #5 | 2.4 | 0.5 | 45.8 | 51.0 | 0.2 |
| | | | " | 120 | #7 | 12.5 | 2.0 | 42.5 | 37.8 | 3.1 |
| | | | | | #2 | 10.9 | 1.3 | 43.0 | 42.4 | 2.4 |
| | | | | 125 | #18 | 19.7 | 3.7 | 39.5 | 31.0 | 5.9[c] |
| | | | " | 140 | #15 | 08.0 | 4.2 | 36.8 | 23.4 | 7.5 |
| | | | | | #19 | 16.3 | 3.7 | 38.2 | 27.8 | 6.6 |
| | | | | | #20 | 27.0 | 4.2 | 38.4 | 26.6 | 7.5 |
| | | | " | 160 | #21 | 25.7 | 5.7 | 38.3 | 21.3 | 9.0 |
| | | | | | #25 | 26.2 | 5.6 | 37.4 | 21.5 | 8.0 |
| | | | | | #27 | 23.8 | 6.0 | 38.2 | 28.5 | 8.4[c] |

a United Catalyst Y-Zeolite, Extrudates
b A Dealuminized Y-Zeolite From Zeochem, Powder
c On-Line

TABLE II

| Example | Catalyst | Feed Sample | Flow Rate (cm³/h) | Temp. (°C) | Sample | MTBE | $i-C_4$ | MeOH | tBA | $H_2O$ |
|---|---|---|---|---|---|---|---|---|---|---|
| | | | | | | ← PRODUCT COMPOSITION (WT%) → | | | | |
| | | F | | | | | | 46.9 | 52.8 | |
| III | Super Acid Alumina[a] | | 25 | 100 | #1 | 1.8 | 0.2 | 46.9 | 50.3 | 0.6 |
| | | | | | #6 | 2.2 | 0.1 | 43.9 | 48.1 | 0.2 |
| | | | | | #7 | 1.2 | 0.2 | 46.8 | 51.7 | -[c] |
| | | | " | 120 | #8 | 1.6 | 0.2 | 46.3 | 51.6 | 0.3 |
| | | | | | #12 | 2.0 | 0.3 | 46.8 | 50.5 | 0.3 |
| | | | | | #14 | 2.1 | 0.3 | 46.2 | 50.2 | 1.1[c] |
| | | | " | 150 | #16 | 6.6 | 1.3 | 45.2 | 44.8 | 2.1 |
| | | | | | #19 | 7.7 | 1.7 | 44.4 | 42.9 | 2.5 |
| | | | | | #20 | 7.6 | 1.9 | 44.1 | 43.6 | 2.6[c] |
| | | F-1 | | | | | | 46.8 | 53.0 | |
| | | | " | 180 | #22 | 14.3 | 6.1 | 42.3 | 32.4 | 4.8 |
| | | | | | #23 | 19.7 | 5.6 | 41.8 | 25.9 | 7.0 |
| | | | " | 210 | #25 | 14.5 | 2.5 | 58.1 | 3.3 | 21.3 |
| | | | | | #29 | 15.9 | 2.6 | 57.3 | 3.1 | 20.8 |
| IV | Super Acid Alumina[b] | | 25 | 180 | #31 | 17.5 | 6.1 | 42.3 | 28.3 | 5.6 |
| | | | | | #32 | 19.8 | 5.3 | 41.6 | 25.5 | 7.7 |

a A High Pore Volume, Super Acid Alumina, 3mm (E-1/8"), AL-4198 (From Harshaw #583A-22-16-6)
b A Super Acid Alumina, 3mm (E-1/8"), Al-3998 (From Harshaw, #583A-22-15-9)
c On-Line Sample

TABLE III

| Example | Catalyst | Feed Sample | Flow Rate (cm³/h) | Temp. (°C) | Sample | MTBE | $i-C_4$ | MeOH | tBA | $H_2O$ |
|---|---|---|---|---|---|---|---|---|---|---|
| | | | | | ← PRODUCT COMPOSITION (WT%) → | | | | | |
| | | F | | | | | | 46.2 | 53.7 | |
| V | Ni-Zeolite[a] | | 25 | 120 | #1 | 0.6 | 0.1 | 46.2 | 52.9 | 0.1 |
| | | | | | #5 | 0.6 | 0.1 | 46.4 | 52.8 | 0.1 |
| | | | | | #6 | 0.8 | - | 46.3 | 52.7 | - [b] |
| | | | " | 150 | #9 | 4.4 | 1.1 | 45.3 | 48.1 | 0.9 |
| | | | | | #11 | 4.2 | 1.2 | 45.2 | 48.4 | 1.0 |
| | | | | | #12 | 4.2 | 1.3 | 45.3 | 48.6 | 0.6[b] |
| | | | " | 180 | #15 | 22.5 | 6.0 | 39.8 | 25.0 | 6.8 |
| | | | | | #16 | 22.4 | 6.4 | 39.6 | 24.5 | 7.0 |

a Zeocat, 7.4% Y Ni On Y-Zeolite, Ni Z6-06-02
b On-Line Sample

TABLE IV

| Example | Catalyst | Feed Sample | Flow Rate $cm^3/h$ | Temp. (°C) | Sample | MTBE | $i\text{-}C_4$ | MeOH | tBA | $H_2O$ |
|---|---|---|---|---|---|---|---|---|---|---|
| | | | | | | ← PRODUCT COMPOSITION (WT%) → | | | | |
| | | 81F | | | | | | 46.3 | 53.6 | |
| VI | Silicate[a] | | 25 | 120 | #2 | 23.4 | 3.9 | 38.0 | 28.1 | 6.6 |
| | | | | | #4 | 23.8 | 3.7 | 38.1 | 28.0 | 6.3 |
| | | | | | #6 | 23.0 | 4.6 | 38.4 | 28.5 | 5.5[b] |
| | | | " | 150 | #8 | 38.3 | 8.2 | 31.4 | 13.0 | 9.1 |
| | | | | | #9 | 37.7 | 6.9 | 32.1 | 13.5 | 9.8 |
| | | | | | #12 | 35.5 | 7.2 | 33.3 | 14.5 | 9.4[b] |
| | | | " | 180 | #14 | 26.4 | 7.8 | 44.4 | 6.4 | 14.8 |
| | | | | | #15 | 24.6 | 6.4 | 46.1 | 6.0 | 16.7[b] |
| | | | | | #18 | 17.9 | 19.3 | 42.0 | 5.9 | 14.9 |
| | | | 100 | 150 | #20 | 19.8 | 6.7 | 39.4 | 28.3 | 5.8 |
| | | | | | #21 | 19.8 | 6.4 | 39.3 | 28.4 | 6.1 |
| | | | | | #24 | 19.9 | 6.0 | 39.6 | 28.5 | 5.8[b] |
| | | 81F[c] | | | | | | 30.2 | 69.8 | |
| | | | 100 | 150 | #27 | 19.2 | 10.3 | 23.5 | 40.2 | 6.9 |
| | | | | | #29 | 19.9 | 9.5 | 23.4 | 40.0 | 7.2 |
| | | | | | #30 | 19.2 | 9.9 | 23.6 | 40.9 | 6.3[b] |
| | | 81F2[c] | | | | | | 30.6 | 69.4 | |
| | | | 25 | 150 | #32 | 36.0 | 12.0 | 17.4 | 25.0 | 9.5 |
| | | | | | #34 | 34.1 | 10.9 | 17.8 | 26.4 | 10.8 |
| | | | | | #36 | 31.9 | 11.2 | 19.0 | 28.4 | 9.6[b] b |

a Silicate, 3 mm (1/16") Extrudates, Type S-115 (Union Carbide, 20% $Al_2O_3$ Binder)
b On-Line Sample
c Use MeOH; tBA, 1:1 Molar Feed

TABLE V

| Example | Catalyst | Feed Sample | Flow Rate ($cm^3/h$) | Temp. (°C) | Sample | ← PRODUCT COMPOSITION (WT%) → MTBE | i-$C_4$ | MeOH | tBA | $H_2O$ |
|---|---|---|---|---|---|---|---|---|---|---|
| VII | Alumina[a] | F | | | | | | 46.6 | 53.3 | |
| | | | 25 | 120 | #1 | 5.0 | 2.0 | 44.2 | 47.3 | 1.5 |
| | | | | | #5 | 4.9 | 1.1 | 44.3 | 47.8 | 1.8 |
| | | | | | #6 | 6.0 | 1.1 | 44.5 | 47.4 | 1.0[b] |
| | | | " | 150 | #7 | 19.2 | 6.9 | 38.2 | 30.4 | 5.3 |
| | | | | | #9 | 20.4 | 4.0 | 39.1 | 30.7 | 5.8 |
| | | | | | #12 | 19.7 | 4.1 | 39.6 | 31.9 | 4.7[b] |
| | | | " | 180 | #13 | 26.4 | 12.3 | 42.6 | 5.2 | 13.4 |
| | | | | | #17 | 28.6 | 9.5 | 42.6 | 5.6 | 13.7 |
| | | | | | #18 | 26.8 | 6.3 | 36.0 | 6.3 | 10.2[b] |

a AERO Extrudate 25 Sample #4110 (American Cyanamid)
b On-Line Sample

Examples VIII to XII illustrate the one-step synthesis of MTBE from TBA and MeOH using acidic clay catalysts, particularly montmorillonite acidic clays.

EXAMPLE VIII

The synthesis was conducted in a tubular reactor 14 mm (0.563") i.d.; 305 mm (12") long, constructed of 316 stainless steel, operated upflow and mounted in a furnace, controllable to + or - 1.0° C and fitted with pumps allowing flow control to < + or - 1 $cm^3$/h. The reactor was also fitted with a pressure regulating device and equipment for monitoring temperature, pressure and flow rate.

The reactor was charged at the beginning of the experiment with 25 $cm^3$ of Harshaw/Filtrol Clay 113. A screen of glass beads was placed at the top and bottom of the reactor to ensure the clay powder would remain in the middle portion.

The catalyst bed was first conditioned overnight by washing with methanol/t-butanol (2:1 mix) at 120° C, 2168 kPa (300 psi) back pressure and a liquid flow rate of 25 $cm^3$/h. The same solution of methanol (1281.6g) plus t-butanol (1482. 4g) was then pumped through the catalyst bed at 25 $cm^3$/h, while the reactor was held at 120° C at a total pressure of 2168 kPa (300 psi). Samples of the product were taken periodically, either by trapping in a dry ice cooled container, or by collecting on-stream (on-line) in a 316 stainless steel bomb. Typical analyses data for samples taken under those conditions are summarized in Table VI.

Catalyst performance at higher temperatures and liquid flow rates were also measured, after reaching equilibrium conditions overnight. Summary data for these three runs are also given in Table VI.

It should be noted that with this catalyst, MTBE is generated in ca. 40% concentration when run at LHSV of 8 (e.g. Sample #21). The operating conditions are moderate (150° C, 2168 kPa (300 psi)) and for Sample #21:

Estimated TBA conversion per pass = 78%

MTBE yield (basis TBA converted) = 81 mole%

TABLE VI

| Example | Catalyst | Feed Sample | Flow Rate (cm³/h) | Temp. (°C) | Sample | PRODUCT COMPOSITION (WT%) MTBE | i-$C_4$ | MeOH | tBA | $H_2O$ |
|---|---|---|---|---|---|---|---|---|---|---|
| | | F | | | | | | 45.5 | 54.2 | |
| VIII | Clay-113[a] | | 25 | 120 | #3 | 28.4 | 1.9 | 35.3 | 25.7 | 8.6 |
| | | | | | #5 | 29.6 | 2.2 | 35.4 | 25.2 | 7.6 |
| | | | | | #6 | 27.5 | 2.3 | 36.5 | 27.5 | 6.2[b] |
| | | | 25 | 150 | #8 | 37.6 | 9.4 | 30.9 | 11.2 | 10.0 |
| | | | | | #9 | 38.1 | 5.0 | 32.5 | 11.9 | 11.5 |
| | | | | | #10 | 38.5 | 3.7 | 33.1 | 12.2 | 11.7 |
| | | | 100 | 150 | #14 | 36.4 | 4.1 | 33.0 | 16.1 | 10.3 |
| | | | | | #15 | 36.7 | 5.3 | 32.4 | 15.5 | 10.0 |
| | | | | | #16 | 37.0 | 5.2 | 32.4 | 15.3 | 10.0 |
| | | F-1 | | | | | | 46.2 | 53.7 | |
| | | | 200 | 150 | #18 | 39.9 | 6.2 | 31.9 | 12.1 | 9.9 |
| | | | | | #19 | 40.0 | 6.2 | 31.9 | 12.1 | 9.8 |
| | | | | | #21 | 40.0 | 6.0 | 31.8 | 12.1 | 9.9 |
| | | F | | | | | | 46.3 | 53.6 | |
| IX | Clay-13[c] | | 25 | 120 | #2 | 29.4 | 2.2 | 36.1 | 25.2 | 7.0 |
| | | | | | #3 | 29.3 | 2.3 | 36.0 | 25.3 | 7.0 |
| | | | | | #6 | 31.9 | 3.2 | 34.5 | 24.6 | 5.8[b] |
| | | | 25 | 150 | #7 | 37.4 | 9.8 | 31.3 | 10.3 | 9.7 |
| | | | | | #11 | 37.7 | 5.4 | 33.6 | 11.2 | 10.7 |
| | | | | | #12 | 36.6 | 6.9 | 33.4 | 11.8 | 9.9[b] |
| | | | 200 | 150 | #13 | 37.9 | 6.9 | 32.8 | 12.2 | 10.0 |
| | | | | | #14 | 37.8 | 6.3 | 33.2 | 12.9 | 9.8 |
| | | | | | #18 | 38.0 | 8.0 | 32.5 | 12.3 | 9.2[b] |

a Harshaw-Filtrol, Clay-113
b On-Line Sample
c Harshaw-Filtrol, Clay-13

EXAMPLE IX TO XII

Using the procedures and analyses methods of Example VIII, these examples illustrate the synthesis of MTBE from methanol plus t-Butanol mix (2:1) employing other acidic clay-catalysts over a range of operating conditions.

a) A sample of Harshaw-Filtrol Clay-13 also gave MTBE in 38% concentration when run at 150 °C and LHSV of 8 (see Table VI, Sample #18).

b) Clay-13, when operated at 150 °C, 2168 kPa (300 psi), with LHSV of 8, will maintain activity over at least 300 hours (see Table VII).

c) A granular form of acidic clay (Harshaw-Filtrol Clay-24) is effective as a MTBE catalyst over the temperature range 80° to 150 °C and LHSV's of 1 to ca. 7 (see Table VIII).

At 120 °C, Example XI, Sample #6, shows:

MTBE Concentration In Product of 44 wt%

tBA Conversion = 77%

MTBE Yield = 91 mole%

At 100 °C, on the other hand, Example XII, Sample #6, shows:

MTBE Concentration In Product Liquid of 44 wt%

tBA Conversion = 74%

MTBE Yield = 94 mole%

Finally at 80 °C, Example XII, Sample #20, shows:

tBA Conversion = 32%

MTBE Yield = 91 mole%

TABLE VIII

| Example | Catalyst | Feed Sample | Flow Rate (cm³/h) | Temp. (°C) | Sample | ← PRODUCT COMPOSITION (WT%) → MTBE | $i-C_4$ | MeOH | tBA | $H_2O$ |
|---|---|---|---|---|---|---|---|---|---|---|
| | | F | | | | | | 46.4 | 53.5 | |
| XI | Clay-24[a] | | 25 | 120 | #2 | 45.3 | 3.7 | 30.0 | 11.7 | 9.2 |
| | | | | | #5 | 43.6 | 3.1 | 31.1 | 12.4 | 9.7 |
| | | | | | #6 | 44.4 | 3.8 | 30.6 | 12.4 | 8.8[b] |
| | | | 25 | 150 | #8 | 36.8 | 6.0 | 33.7 | 10.8 | 10.7 |
| | | | | | #10 | 37.1 | 4.2 | 34.4 | 11.1 | 11.1 |
| | | | | | #12 | 36.3 | 7.5 | 33.5 | 10.6 | 9.9[b] |
| | | | 120 | 150 | #13 | 38.6 | 6.2 | 33.4 | 11.1 | 10.2 |
| | | | | | #17 | 38.3 | 7.0 | 33.2 | 10.9 | 10.1 |
| | | | | | #18 | 38.1 | 8.2 | 32.7 | 10.8 | 9.6[b] |
| | | F | | | | | | 46.3 | 53.6 | |
| XII | Clay-24[a] | | 25 | 100 | #1 | 44.2 | 2.8 | 30.3 | 13.7 | 9.0 |
| | | | | | #5 | 43.9 | 1.8 | 30.9 | 14.2 | 9.2 |
| | | | | | #6 | 44.0 | 2.6 | 30.8 | 14.1 | 8.5[b] |
| | | | 120 | 100 | #7 | 30.1 | 2.3 | 35.8 | 25.4 | 6.4 |
| | | | | | #11 | 30.0 | 2.1 | 36.0 | 25.7 | 6.3 |
| | | | | | #12 | 29.7 | 2.7 | 35.9 | 25.6 | 6.1[b] |
| | | | 25 | 80 | #15 | 18.0 | 1.6 | 40.3 | 36.6 | 3.5 |
| | | | | | #19 | 17.9 | 1.3 | 40.3 | 36.9 | 3.5 |
| | | | | | #20 | 18.2 | 1.4 | 40.2 | 36.6 | 3.6[b] |

a Harshaw-Filtrol, Clay-24
b One-line Sample

TABLE VII

| Example | Catalyst | Sample | MTBE | i-$C_4$ | MeOH | TBA | $H_2O$ | Stream (Days) |
|---|---|---|---|---|---|---|---|---|
| X | Clay 13[a] | #1 | 39.3 | 5.6 | 32.8 | 11.7 | 10.3 | 1 |
| | | #2 | 39.2 | 6.7 | 32.5 | 11.3 | 10.1 | 2 |
| | | #3 | 39.6 | 6.2 | 32.6 | 11.5 | 10.0 | 3 |
| | | #4 | 39.5 | 6.0 | 32.9 | 11.6 | 9.9 | 4 |
| | | #5 | 38.9 | 5.9 | 33.2 | 12.0 | 9.9 | 5 |
| | | #6 | 39.7 | 2.5 | 34.6 | 12.2 | 10.9 | 7 |
| | | #7 | 39.3 | 6.1 | 33.4 | 11.1 | 10.1 | 8 |
| | | #8 | 38.2 | 6.6 | 32.9 | 12.5 | 9.6 | 9 |
| | | #9 | 38.0 | 6.2 | 33.2 | 13.0 | 9.6 | 10 |
| | | #10 | 37.9 | 6.4 | 33.2 | 12.8 | 9.6 | 11 |
| | | #11 | 38.1 | 2.0 | 35.7 | 13.3 | 10.9 | 14 |
| | | #12 | 35.7 | 5.2 | 34.4 | 15.4 | 9.1 | 14 |
| | | #13 | 33.9 | 6.0 | 34.5 | 16.8 | 8.6 | 15 |

a Run at 200 $cm^3/h$, 150°C, 2168 kPa (300 psi)

## Claims

1. A method for producing methyl t-butyl ether wherein t-butanol is reacted with methanol in the presence of a catalyst characterised in that:
   a) the catalyst is an acidic alumina or acidic silica, alone or combined with a zeolite or with an acidic clay material;

b) and in that the reaction is conducted in a single step by continuously contacting from 0.1 to 10 moles of methanol per mole of t-butanol with said catalyst at a, temperature of 20 to 250° C and a pressure of atmospheric to 6995 kPa (0 to 1000 psig)

**2.** A method according to Claim 1 characterised in that the alumina catalyst is a super acid alumina having both Lewis and Bronsted Acid Sites and a surface area of greater than 10 $m^2/g$.

**3.** A method according to Claim 1 characterised in that the zeolite catalyst is a silica-rich zeolite.

**4.** A method according to Claim 1 characterised in that the zeolite catalyst is zeolite-Y, dealuminized zeolite-Y or silicalite.

**5.** A method according to Claim 1 characterised in that the acidic clay catalyst is an acidic smectite clay.

**6.** A method according to Claim 5 characterised in that the acidic smectite clay is a montmorillonite clay.

**7.** A method according to any one of Claims 1 to 6 characterised in that the catalyst is in the form of extrudates, powders or granules.

## Patentansprüche

**1.** Verfahren zur Herstellung von Methyl-tert.-butylether, bei dem tert.-Butanol in Gegenwart eines Katalysators mit Methanol zur Reaktion gebracht wird, dadurch gekennzeichnet, daß:

a) der Katalysator ein saures Aluminiumoxid oder saures Siliziumdioxid ist, allein oder kombiniert mit einem Zeolithen oder mit einem sauren Ton-Material;

b) und daß die Reaktion in einer einzigen Stufe durchgeführt wird, indem von 0,1 bis 10 Mol Methanol pro Mol tert.-Butanol kontinuierlich mit besagtem Katalysator bei einer Temperatur von 20 bis 250° C und einem Druck von atmosphärisch bis 6995 kPa (0 bis 100 psig) in Kontakt gebracht werden.

**2.** Verfahren nach Anspruch 1, dadurch gekennzeichnet, daß der Aluminiumoxid-Katalysator ein superazides Aluminiumoxid mit sowohl Lewis- als auch Bronsted-Säurestellen und einer Oberfläche von mehr als 10 $m^2/g$ ist.

**3.** Verfahren nach Anspruch 1, dadurch gekennzeichnet, daß der Zeolith-Katalysator ein siliziumdioxidreicher Zeolith ist.

**4.** Verfahren nach Anspruch 1, dadurch gekennzeichnet, daß der Zeolith-Katalysator Zeolith-Y, dealumierter Zeolith-Y oder Silicalite ist.

**5.** Verfahren nach Anspruch 1, dadurch gekennzeichnet, daß der saure Tonkatalysator ein saurer Smektit-Ton ist.

**6.** Verfahren nach Anspruch 5, dadurch gekennzeichnet, daß der saure Smektit-Ton ein Montmorillonit-Ton ist.

**7.** Verfahren nach einem der Ansprüche 1 bis 6, dadurch gekennzeichnet, daß der Katalysator in Form von Extrudaten, Pulvern oder Körnern vorliegt.

## Revendications

**1.** Procédé de production de l'oxyde de méthyle et de butyle tertiaire dans lequel on fait réagir le t-butanol avec le méthanol, en présence d'un catalyseur, procédé caractérisé par le fait que:

a) le catalyseur est une alumine activée par un acide ou une silice activée par un acide, seule ou combinée avec une zéolite ou avec un matériau du type argile activée par un acide;

b) et par le fait que la réaction s'effectue en une seule étape par mise en contact continue de 0,1 à 10 moles de méthanol par mole de t-butanol avec ledit catalyseur à une température allant de 20 à 250° C et sous une pression allant de la pression atmosphérique à 6895 kPa (0 à 1000 psig).

2. Procédé selon la revendication 1, caractérisé par le fait que le catalyseur à base d'alumine est une alumine super acide présentant à la fois des sites acides au sens de Lewis et des sites acides au sens de Bronsted et ayant une surface spécifique supérieure à 10 $m^2/g$.

3. Procédé selon la revendication 1, caractérisé par le fait que le catalyseur à base de zéolite est une zéolite riche en silice.

4. Procédé selon la revendication 1, caractérisé par le fait que le catalyseur à base de zéolite est de la zéolite Y, de la zéolite Y désaluminisée, ou de la silicalite.

5. Procédé selon la revendication 1, caractérisé par le fait que le catalyseur à base d'argile activée par un acide est une argile du type smectite activée par un acide.

6. Procédé selon la revendication 5, caractérisé par le fait que l'argile du type smectite activée par un acide est une argile du type montmorillonite.

7. Procédé selon l'une quelconque des revendications 1 à 6, caractérisé par le fait que les catalyseurs se présentent sous forme d'extrudats, de poudres ou de granulés.